(19) Europäisches Patentamt
European Patent Office
Office européen des brevets

(11) EP 3 199 941 B1

(12) **FASCICULE DE BREVET EUROPEEN**

(45) Date de publication et mention
de la délivrance du brevet:
**20.10.2021 Bulletin 2021/42**

(51) Int Cl.:
**G01N 21/45** *(2006.01)* **G01N 15/14** *(2006.01)*

(21) Numéro de dépôt: **17152837.5**

(22) Date de dépôt: **24.01.2017**

(54) **PROCÉDÉ D'OBSERVATION D'UN ÉCHANTILLON PAR IMAGERIE SANS LENTILLE**

VERFAHREN ZUR BEOBACHTUNG EINER PROBE MITTELS BILDGEBUNGSSYSTEM OHNE LINSE

METHOD FOR OBSERVING A SAMPLE BY LENS-FREE IMAGING

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **25.01.2016 FR 1650575**

(43) Date de publication de la demande:
**02.08.2017 Bulletin 2017/31**

(73) Titulaire: **Commissariat à l'énergie atomique et aux énergies alternatives
75015 Paris (FR)**

(72) Inventeurs:
• **MOREL, Sophie
59500 DOUAI (FR)**
• **ALLIER, Cédric
38000 GRENOBLE (FR)**

(74) Mandataire: **INNOV-GROUP
310, avenue Berthelot
69372 Lyon Cedex 08 (FR)**

(56) Documents cités:
WO-A1-2008/090330    WO-A2-2012/094523
US-A1- 2012 142 086    US-A1- 2012 218 379
US-A1- 2014 248 713

• DAN ZHU ET AL: "Recent progress in tissue optical clearing", LASER & PHOTONICS REVIEWS, vol. 7, no. 5, 31 janvier 2013 (2013-01-31), pages 732-757, XP055217356, ISSN: 1863-8880, DOI: 10.1002/lpor.201200056
• ALLIER C P ET AL: "Lensfree microscopy: A new framework for the imaging of viruses, bacteria, cells and tissue", 2015 IEEE INTERNATIONAL ELECTRON DEVICES MEETING (IEDM), IEEE, 7 décembre 2015 (2015-12-07), XP032865548, DOI: 10.1109/IEDM.2015.7409690
• ZHERNOVAYA OLGA S ET AL: "Study of optical clearing of blood by immersion method", DYNAMICS AND FLUCTUATIONS IN BIOMEDICAL PHOTONICS VIII, SPIE, 1000 20TH ST. BELLINGHAM WA 98225-6705 USA, vol. 7898, no. 1, 10 février 2011 (2011-02-10), pages 1-8, XP060006758, DOI: 10.1117/12.879822
• A. Greenbaum ET AL: "Wide-field computational imaging of pathology slides using lens-free on-chip microscopy", Science Translational Medicine, vol. 6, no. 267, 17 December 2014 (2014-12-17), pages 267ra175-267ra175, XP055259215, US ISSN: 1946-6234, DOI: 10.1126/scitranslmed.3009850

EP 3 199 941 B1

**Description**

**DOMAINE TECHNIQUE**

Le domaine technique de l'invention est lié à l'observation d'un échantillon, en particulier une lamelle de tissu biologique, par imagerie sans lentille. L'échantillon est un tissu biologique, l'observation étant réalisée à des fins d'analyse histologique.

**ART ANTERIEUR**

L'observation d'échantillons, et en particulier des échantillons biologiques, par imagerie sans lentille connaît un développement important depuis ces dix dernières années. Cette technique permet d'observer un échantillon en le disposant entre une source de lumière et un capteur d'image, sans disposer de lentille de grossissement optique entre l'échantillon et le capteur d'image. Ainsi, le capteur d'image collecte une image de l'onde lumineuse transmise par l'échantillon.

Cette image est formée de figures d'interférence entre l'onde lumineuse émise par la source de lumière et transmise par l'échantillon, et des ondes de diffraction, résultant de la diffraction par l'échantillon de l'onde lumineuse émise par la source de lumière. Ces figures d'interférences sont parfois dénommées figures de diffraction, ou désignées par le terme anglais « diffraction pattern ».

Le document WO2008090330 décrit un dispositif permettant l'observation d'échantillons biologiques, en l'occurrence des cellules, par imagerie sans lentille. Le dispositif permet d'associer, à chaque cellule, une figure d'interférence dont la morphologie permet d'identifier le type de cellule. L'imagerie sans lentille apparaît alors comme une alternative simple, et peu onéreuse, à un microscope classique. De plus, son champ d'observation est nettement plus important que ne peut l'être celui d'un microscope. On comprend alors que les perspectives d'application liées à cette technologie sont importantes. Le document US2012/142086 décrit une application de l'imagerie sans lentille à l'observation de cellules baignant dans un milieu aqueux. Un liquide peut s'étendre entre les cellules observées et des microlentilles d'un capteur d'image.

D'une façon générale, l'image formée sur le capteur d'image, comportant des figures d'interférence, peut être traitée par un algorithme de reconstruction holographique, de manière à estimer des propriétés optiques de l'échantillon. De tels algorithmes sont bien connus dans le domaine de la reconstruction holographique. Pour cela, la distance entre l'échantillon et le capteur d'image étant connue, on applique un algorithme de propagation, prenant en compte cette distance, ainsi que la longueur d'onde de l'onde lumineuse émise par la source de lumière. On peut alors reconstituer une image d'une propriété optique de l'échantillon. Un algorithme de reconstruction numérique est par exemple décrit dans US2012/0218379. Le document WO2012/094523 décrit également le recours à des reconstructions holographiques pour obtenir des images du module ou de la phase d'un échantillon examiné, de façon à en obtenir une représentation tridimensionnelle.

On a récemment démontré l'intérêt de l'imagerie sans lentille pour l'analyse histologique de prélèvements tissulaires et tumoraux prenant la forme d'une tranche fine, ou lamelle, déposée sur un support transparent. Le large champ d'observation conféré par l'imagerie sans lentille, couplé à son faible coût constituent des avantages décisifs par rapport à l'usage d'un microscope classique. De plus, le recours à des algorithmes de reconstruction holographique permet de former une image d'absorption ou une image de phase de l'échantillon observé, d'une qualité suffisante pour effectuer une analyse histologique fiable.

La publication Greenbaum A, « wide-field computational imaging of pathology slides using lensfree on-chip microscopy », Sci. Transl. Med 6, 267ra175 (2014), décrit par exemple l'application de l'imagerie sans lentille à des lames d'anatomo-pathologie formées à en prélevant différents types de tissus biologiques. Il en est de même quant à la publication Luo W. « Synthetic aperturebased on-chip microscopy », Light : Science & Applications (2015) 4, e261. Dans cette publication, une huile de couplage optique est appliquée entre le capteur d'image et l'échantillon de façon à améliorer le couplage optique. Cependant, le temps nécessaire à l'obtention d'une image d'un échantillon de 1 mm$^2$ dépasse 45 minutes, ce qui est encore trop important, et peu compatible avec une utilisation en routine.

Par ailleurs, l'utilisation d'agents dits éclaircissants, usuellement désignés par le terme anglosaxon "clearing agents", a été décrite pour observer des tissus biologiques, et plus précisément pour permettre une meilleure observation de ces tissus en profondeur. On en trouve des exemples dans les publications Zhu Dan "Recent progress in tissue optical clearing", Laser Photonics Rev. 7, No 5, 732-757 (2013) ainsi que dans la publication Zhernovaya Olga, "Study of optical clearing of blood by immersion method", Dynamics and fluctuations in biomedical photonics VIII, proc of SPIE Vol. 7898.

Les inventeurs proposent un procédé d'observation d'un échantillon, et en particulier un prélèvement tissulaire, par imagerie sans lentille, mettant en œuvre des procédés de reconstruction holographiques, permettant la formation d'une image plus nette. Ce procédé est particulièrement adapté à l'observation de tissus biologiques.

## EXPOSE DE L'INVENTION

Un premier objet de l'invention est un procédé d'observation d'un échantillon selon la revendication 1, comportant les étapes suivantes :

i) illumination dudit échantillon à l'aide d'une source de lumière, apte à émettre une onde lumineuse incidente se propageant vers l'échantillon ;
ii) acquisition, à l'aide d'un capteur d'image s'étendant selon un plan de détection, d'au moins une image de l'échantillon, l'échantillon étant disposé entre la source de lumière et le capteur d'image, chaque image étant représentative d'une onde lumineuse transmise par l'échantillon sous l'effet de ladite illumination ;
iii) application d'un opérateur de propagation à l'image acquise, lors de l'étape ii), de façon à former une image représentative de ladite onde lumineuse transmise par l'échantillon, dans un plan passant par ledit échantillon ;

le procédé étant caractérisé en ce qu'il comprend également, préalablement à l'étape ii), l'application d'un liquide d'imprégnation sur l'échantillon, de telle sorte que l'échantillon s'imprègne dudit liquide d'imprégnation, ledit liquide d'imprégnation ayant un indice de réfraction strictement supérieur à 1.

L'onde lumineuse transmise par l'échantillon correspond à une onde lumineuse à laquelle est exposé le capteur d'image.

L'imprégnation de l'échantillon peut être réalisée avant l'étape i) ou entre les étapes i) et ii).

De préférence, l'image formée lors de l'étape iii), représentative de l'onde lumineuse transmise par l'échantillon, c'est-à-dire de l'onde lumineuse à laquelle est exposé le capteur d'image, est établie à partir de la phase, dans le plan de l'échantillon, de ladite onde lumineuse transmise par l'échantillon. Ainsi, l'étape iii) permet de déterminer la phase de cette onde lumineuse dans un plan passant par l'échantillon. Le plan de passant par l'échantillon peut être un plan selon lequel s'étend l'échantillon. Il est de préférence sensiblement parallèle au plan de détection. Le procédé peut comprendre l'application d'un filtre passe-haut à l'image formée lors de l'étape iii).

De préférence, le liquide d'imprégnation a un indice de réfraction compris entre 1.2 et 1.8 ou entre 1.3 et 1.7.

Selon un mode de réalisation, ledit liquide d'imprégnation s'étend de l'échantillon jusqu'au capteur d'image. Le capteur d'image comprend une pluralité de pixels, et le liquide d'imprégnation s'étend de l'échantillon jusqu'à ces pixels. Chaque pixel peut être couplé à une microlentille de focalisation, auquel cas le liquide d'imprégnation peut s'étendre de l'échantillon jusqu'à chaque microlentille. Selon ce mode de réalisation, le liquide d'imprégnation peut être appliqué sur l'échantillon ou sur le capteur d'image, après quoi l'échantillon est rapproché du capteur d'image, jusqu'à ce que le liquide d'imprégnation s'étende de l'échantillon jusqu'au capteur d'image.

Selon un mode de réalisation préféré, une lame transparente est disposée entre l'échantillon et le capteur d'image, le liquide d'imprégnation s'étendant entre l'échantillon et ladite lame transparente.

L'échantillon peut notamment être une lamelle de tissu biologique, en particulier apte à la réalisation d'analyses histologiques. Il peut en particulier être déposé sur une lame support transparente.

De préférence, l'échantillon est rendu transparent ou translucide après l'application du liquide d'imprégnation. L'application du liquide d'imprégnation a alors pour fonction d'améliorer la transparence de l'échantillon.

Selon un mode de réalisation, l'étape ii) comporte la formation d'une pluralité d'images, chaque image étant acquise dans une bande spectrale différente l'une de l'autre l'étape iii) comportant les sous-étapes suivantes :

a) détermination, à partir de chaque image acquise selon une bande spectrale, d'une amplitude complexe initiale de l'onde lumineuse transmise, selon ladite bande spectrale, dans ledit plan de détection ;
b) à partir de chaque amplitude complexe établie dans ledit plan de détection, selon une bande spectrale, détermination d'une amplitude complexe de l'onde transmise, selon chaque bande spectrale, dans le plan selon lequel s'étend l'échantillon ;
c) combinaison d'une pluralité d'amplitudes complexes déterminées lors de la sous-étape b), à différentes bandes spectrales, pour calculer une fonction de pondération, dans le plan de l'échantillon ;
d) projection de ladite fonction de pondération dans le plan de détection de façon à obtenir, pour chaque bande spectrale, une fonction de pondération dans ledit plan de détection ;
e) mise à jour de chaque amplitude complexe de l'onde lumineuse transmise, selon chaque bande spectrale, dans le plan de détection, à l'aide de de ladite fonction de pondération obtenue, dans ladite bande spectrale, au cours de la sous-étape d) ;
f) répétition éventuelle des sous-étapes b) à e) jusqu'à l'atteinte d'un critère d'arrêt.

Le procédé peut alors comporter une sous-étape g) de formation d'une image représentative de l'argument, dans le plan de l'échantillon, de l'amplitude complexe de l'onde transmise par l'échantillon selon au moins une bande spectrale. Cette image peut être représentative d'une combinaison, par exemple une somme ou une moyenne, de l'argument de l'amplitude complexe, dans le plan de l'échantillon, de ladite onde transmise, selon différentes bandes spectrales.

De préférence, aucune optique de grossissement n'est disposée entre l'échantillon et le capteur d'image.

## FIGURES

La figure 1A représente un premier exemple de dispositif pour la mise en œuvre de l'invention, l'échantillon analysé étant une lame d'anatomo-pathologie. Les figures 1B et 1C représentent différentes configurations de l'échantillon.

La figure 2A représente le plan de détection, sur lequel une image est formée, ainsi que le plan selon lequel s'étend l'échantillon. Cette figure illustre également les liens entre les principales grandeurs mises en œuvre dans un algorithme de reconstruction avantageux.

La figure 2B représente un logigramme décrivant l'enchaînement des principales étapes de l'algorithme de reconstruction avantageux illustré sur la figure 2A.

La figure 3A représente une image d'un échantillon dit de référence, acquise par le capteur d'image. L'échantillon de référence est un échantillon configuré selon l'art antérieur. La figure 3B représente une image d'un échantillon dit de test, acquise par le capteur d'image.

L'échantillon de référence et l'échantillon de test sont formés d'une lame de tissu prélevé dans un intestin. L'échantillon de test est configuré conformément à un mode de réalisation de l'invention.

Les figures 4A et 4B représentent respectivement une image du module et une image dite de phase d'une onde lumineuse transmise par l'échantillon de référence. Les figures 4C et 4D représentent respectivement un détail des figures 4A et 4B.

Les figures 5A et 5B représentent respectivement une image du module et une image dite de phase d'une onde lumineuse transmise par l'échantillon de test. Les figures 5C et 5D représentent respectivement un détail des figures 5A et 5B. Les figures 5E et 5F sont des vues de l'échantillon de test obtenues selon une modalité de microscopie classique, le champ d'observation correspondant respectivement aux détails illustrés sur les figures 5A et 5B.

La figure 6A représente une image, dite de phase, de l'échantillon de test. La figure 6B est une image obtenue de ce même échantillon selon une modalité de microscopie classique.

## EXPOSE DE MODES DE REALISATION PARTICULIERS

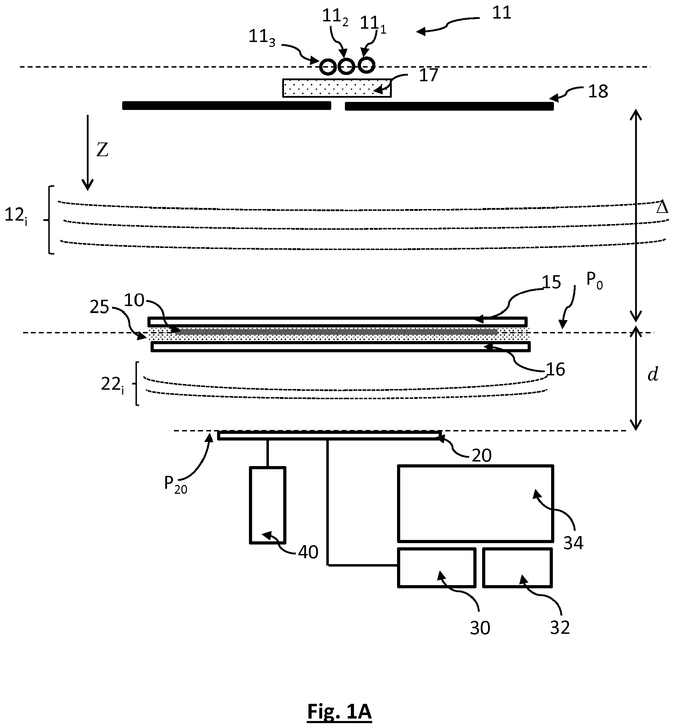

La figure 1A représente un exemple de dispositif objet de l'invention. Une source de lumière 11 est apte à émettre une onde lumineuse 12, dite onde lumineuse incidente, se propageant en direction d'un échantillon 10, selon un axe de propagation Z.

L'échantillon 10 peut être un échantillon biologique que l'on souhaite caractériser. Il peut notamment s'agir d'une lame de tissu destinée à une analyse histologique, ou lame d'anatomopathologie, comportant une fine épaisseur de tissu déposée sur une lame transparente 15. Par fine épaisseur, on entend une épaisseur de préférence inférieure à 100 $\mu$m, et de préférence inférieure à 10 $\mu$m, typiquement quelques micromètres. Un tel échantillon est représenté sur la figure 1A. L'échantillon s'étend selon un plan $P_0$, dit plan de l'échantillon, perpendiculaire à l'axe de propagation Z.

Cette lame de tissu est obtenue selon des procédés de préparation connus, à partir d'un échantillon tissulaire prélevé par biopsie ou frottis, puis préparé de façon à se présenter sous la forme d'une fine épaisseur déposée sur une lame transparente, cette dernière servant de support. De tels procédés sont connus dans le domaine de l'histologie. Ils comportent par exemple une découpe d'un tissu congelé, ou une inclusion d'un tissu prélevé dans une matrice de paraffine.

La distance $\Delta$ entre la source de lumière et l'échantillon est de préférence supérieure à 1 cm. Elle est de préférence comprise entre 2 et 30 cm. De préférence, la source de lumière, vue par l'échantillon, est considérée comme ponctuelle. Cela signifie que son diamètre (ou sa diagonale) est préférentiellement inférieur au dixième, mieux au centième de la distance entre l'échantillon et la source de lumière. Ainsi, de préférence, la lumière parvient à l'échantillon sous la forme d'ondes planes, ou pouvant être considérées comme telles.

Dans l'exemple de la figure 1A, la source de lumière 11 comporte trois diodes électroluminescentes (LED, acronyme du terme anglais Light Emitting Diode) $11_1$, $11_2$ et $11_3$ émettant respectivement dans une première bande spectrale $\lambda_1$ = 450nm - 465 nm, une deuxième bande spectrale $\lambda_2$ = 520nm - 535 nm et une troisième bande spectrale $\lambda_3$ = 620nm - 630 nm. Ces trois diodes électroluminescentes constituent des sources élémentaires de la source de lumière 11. Dans cet exemple, la source de lumière est une diode électroluminescente (LED, acronyme de Light Emitting Diode) fournie par CREE sous la référence XLamp MCE. Les trois diodes électroluminescentes élémentaires $11_1$, $11_2$ et $11_3$ la composant sont activées simultanément. De façon alternative, ces diodes électroluminescentes peuvent être activées successivement.

La source de lumière 11 est de préférence ponctuelle. Elle peut notamment comprendre un diaphragme 18, ou filtre spatial. L'ouverture du diaphragme est typiquement comprise entre 5 $\mu$m et 1mm, de préférence entre 50 $\mu$m et 500 $\mu$m. Dans cet exemple, le diaphragme est fourni par Thorlabs sous la référence P150S et son diamètre est de 150 $\mu$m. Le diaphragme peut être remplacé par une fibre optique, dont une première extrémité est placée face à la source de lumière 11 et dont une deuxième extrémité est placée face à l'échantillon 10.

Le dispositif comporte de préférence un diffuseur 17, disposé entre la source de lumière 11 et le diaphragme 18. L'usage d'un tel diffuseur permet de s'affranchir de contraintes de centrage de la source de lumière 11 par rapport à l'ouverture du diaphragme 18. La fonction d'un tel diffuseur est de répartir le faisceau lumineux, produit par une source de lumière élémentaire $11_i$, ($1 \leq i \leq 3$) selon un cône d'angle a, a étant égal à 40° dans le cas présent. De préférence, l'angle de diffusion $\alpha$ varie entre 10° et 80°. Dans cet exemple, le diffuseur est fourni par Luminit sous la référence Luminit LSD 40°.

L'échantillon 10 est disposé entre la source de lumière 11 et un capteur d'image 20. Ce dernier s'étend de préférence parallèlement, ou sensiblement parallèlement à la lame transparente 15 supportant l'échantillon. Le terme sensiblement parallèlement signifie que les deux éléments peuvent ne pas être rigoureusement parallèles, une tolérance angulaire de quelques degrés, inférieure à 20° ou 10° étant admise.

Le capteur d'image 20 est apte à former une image selon un plan de détection $P_{20}$. Dans l'exemple représenté, il s'agit d'un capteur d'image comportant une matrice de pixels, de type CCD ou un CMOS. Les CMOS sont les capteurs préférés car la taille des pixels est plus faible, ce qui permet d'acquérir des images dont la résolution spatiale est plus favorable. Dans cet exemple, le capteur d'image est un capteur CMOS commercialisé par Omnivision sous la référence OV5647. Il s'agit d'un capteur CMOS RGB, comprenant 2592 x 1944 pixels carrés de taille 1.4 $\mu$m. La surface utile du capteur est de 3.6 x 2.7 $mm^2$. Les capteurs dont la taille des pixels est inférieure à 3 $\mu$m sont préférés, afin d'améliorer la résolution spatiale de l'image. Le plan de détection $P_{20}$ s'étend de préférence perpendiculairement à l'axe de propagation Z de l'onde lumineuse incidente 12.

Le capteur d'image comporte un filtre de Bayer, de telle sorte que chaque pixel est sensible à une bande spectrale choisie parmi le bleu, le rouge ou le vert. Ainsi, lorsque l'échantillon est exposé à la source de lumière 11, le capteur d'image 20 acquiert une image $I$ pouvant être décomposée en :

- une première image $I_1$ dans la première bande spectrale $\lambda_1$ d'émission de la première diode électroluminescente $11_1$, cette image étant formée à partir des pixels exposés à une longueur d'onde transmise par le filtre bleu du filtre de Bayer;
- une deuxième image $I_2$ dans la deuxième bande spectrale $\lambda_2$ d'émission de la deuxième diode électroluminescente $11_2$, cette image étant formée à partir des pixels exposés à une longueur d'onde transmise par le filtre vert du filtre de Bayer;
- une troisième image $I_3$ dans la troisième bande spectrale $\lambda_3$ d'émission de la troisième diode électroluminescente $11_3$, cette image étant formée à partir des pixels exposés à une longueur d'onde transmise par le filtre rouge du filtre de Bayer.

D'une façon générale, selon ce mode de réalisation, le capteur d'image 20 permet l'acquisition d'images $I_i$ de l'échantillon 10 dans différentes bandes spectrales $\lambda_i$. Chaque image $I_i$ est représentative d'une onde lumineuse $22_i$ transmise par l'échantillon 10, et à laquelle est exposée le capteur d'image, dite onde lumineuse d'exposition, dans chaque bande spectrale $\lambda_i$. De préférence, il n'y a pas de recouvrement entre les différentes bandes spectrales ; un recouvrement négligeable, par exemple concernant moins de 25%, mieux moins de 10% de l'intensité lumineuse émise, est toutefois envisageable.

D'autres configurations sont possibles, par exemple l'utilisation d'un capteur d'image monochrome, acquérant une image $I_i$ de l'échantillon lorsque ce dernier est successivement illuminé par une onde incidente $12_i$, et cela dans différentes bandes spectrales $\lambda_i$. Chaque onde incidente $12_i$ peut être émise par une source de lumière $11_i$ émettant dans une desdites bandes spectrales, ou par une source de lumière blanche filtrée par un filtre optique dont la bande passante correspond à ladite bande spectrale $\lambda_i$.

La distance d entre l'échantillon 10 et la matrice de pixels du capteur d'image 20 est, dans cet exemple, égale à 300 $\mu$m. D'une manière générale, et cela quel que soit le mode de réalisation, la distance d entre l'échantillon et les pixels du capteur d'image est préférentiellement comprise entre 50 $\mu$m et 2 cm, de préférence comprise entre 100 $\mu$m et 2 mm.

On remarque l'absence d'optique de grossissement entre le capteur d'image 20 et l'échantillon 10. Cela n'empêche pas la présence éventuelle de microlentilles de focalisation au niveau de chaque pixel du capteur d'image 20, ces dernières n'ayant pas de fonction de grandissement de l'image acquise par le capteur d'image.

Sous l'effet de l'onde lumineuse incidente $12_i$, l'échantillon 10 peut engendrer une onde diffractée, susceptible de produire, au niveau du plan de détection $P_{20}$, des interférences, en particulier avec une partie de l'onde lumineuse incidente $12_i$ transmise par l'échantillon. Par ailleurs, l'échantillon peut absorber une partie de l'onde lumineuse incidente

EP 3 199 941 B1

$12_i$. Ainsi, l'onde lumineuse $22_i$, dans une bande spectrale $\lambda_i$, transmise par l'échantillon, dite onde lumineuse d'exposition, et à laquelle est exposé le capteur d'image 20, peut comprendre :

- une composante résultant de la diffraction de l'onde lumineuse incidente $12_i$ par l'échantillon ;
- une composante résultant de l'absorption de l'onde lumineuse incidente $12_i$ dans l'échantillon.

Un processeur 30, par exemple un microprocesseur, est apte à traiter chaque image acquise par le capteur d'image 20. En particulier, le processeur est un microprocesseur relié à une mémoire programmable 32 dans laquelle est stockée une séquence d'instructions pour effectuer les opérations de traitement d'images et de calculs décrites dans cette description. Le processeur peut être couplé à un écran 34 permettant l'affichage d'images acquises par le capteur d'image 20 ou calculées par le processeur 30.

Le dispositif comporte également un actionneur 40 permettant un déplacement relatif de l'échantillon 10 par rapport au capteur d'image 20, ce déplacement étant de préférence réalisé selon une direction perpendiculaire à l'axe de propagation Z, c'est-à-dire parallèlement au plan de détection $P_{20}$. Un tel déplacement permet d'effectuer un balayage d'un échantillon de grande surface, typiquement de quelques cm². Le champ d'observation du capteur d'image est voisin de la taille du capteur CMOS, soit 3.6 x 2.7 mm soit 9.7 mm², du fait de la faible distance d entre l'échantillon et le capteur. Il est donc nécessaire de déplacer le capteur d'image 20 relativement à l'échantillon 10 pour effectuer une observation complète de ce dernier.

Un aspect important de l'invention porte sur la configuration de l'échantillon 10. Préalablement à l'acquisition d'images par le capteur d'image, un liquide d'imprégnation 25 est déposé sur cet échantillon. Ce liquide d'imprégnation 25 est un liquide ou un gel dont l'indice de réfraction est strictement supérieur à 1, et de préférence supérieur à 1.2 Avantageusement, cet indice de réfraction est proche de l'indice de réfraction de l'échantillon, ou compris entre l'indice de réfraction de l'échantillon et l'indice de réfraction du capteur d'image, en particulier des microlentilles de focalisation associées à chaque pixel. Par indice de réfraction proche de celui de l'échantillon, on entend compris entre +50% et -50% ou +30% et -30% de l'indice de réfraction de l'échantillon. L'indice de réfraction du liquide d'imprégnation est par exemple compris entre 1 et 2. Avantageusement, il est compris entre 1.2 et 1.8. Lorsque l'échantillon 10 comporte un tissu congelé, l'indice de réfraction est de préférence compris entre 1.3 et 1.5. Lorsque l'échantillon 10 est un tissu inclus dans une matrice de paraffine, l'indice de réfraction est de préférence voisin de 1.5, par exemple compris entre 1.4 et 1.7.

Le liquide d'imprégnation 25 est placé au contact de l'échantillon 10 et ce dernier, du fait de sa porosité, s'en imprègne. L'échantillon devient alors transparent ou translucide sous l'effet de cette imprégnation. Les inventeurs ont constaté qu'une telle configuration permettait d'obtenir une image de bonne qualité de l'échantillon, comme décrit ultérieurement, en lien avec les essais expérimentaux réalisés. Le liquide d'imprégnation peut être choisi parmi un agent connu pour éclaircir les tissus. Il peut par exemple s'agir de BABB, acronyme de « Benzyl alcohol / Benzyl Benzoate », agent éclaircicant connu de l'homme du métier. Il peut également s'agir de glycérol, d'éthanol, ou de DMSO, acronyme de Diméthylsulfoxyde. D'autres agents connus pour éclaircir les tissus, dénommés par le terme anglais « clearing agents », sont mentionnés dans la publication Zhu D « Recent Progress in Tissue Optical Clearing » Laser&Photonics Reviews. 2013 7(5) :732-757, ou dans les autres publications citées en lien avec l'art antérieur.

Comme illustré sur la figure 1A, le liquide d'imprégnation 25 peut remplir un espace comprenant l'échantillon 10, cet espace étant délimité par la lame 15 précédemment décrite, dite lame support, et une autre lame, dite lame de confinement 16, s'étendant de référence parallèlement, ou sensiblement parallèlement, à la lame support 15 et au plan de détection $P_{20}$. Cette configuration permet de former des interfaces planes entre les différents milieux traversés par l'onde lumineuse transmise par l'échantillon jusqu'au capteur d'image 20.

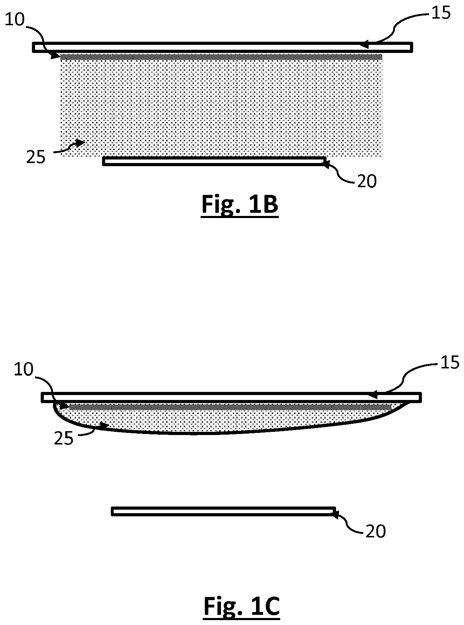

La lame de confinement 16 est optionnelle et peut être omise, comme représenté sur la figure 1B. Dans cette configuration, le liquide d'imprégnation 25 s'étend entre l'échantillon 10 et le capteur d'images 20. Dans cet exemple de réalisation, le liquide d'imprégnation s'étend jusqu'aux microlentilles de focalisation disposées face à chaque pixel. Cela permet d'assurer une adaptation de l'indice de réfraction depuis l'échantillon jusqu'à chaque pixel.

Selon le mode de réalisation représenté sur la figure 1C, le liquide d'imprégnation est disposé au contact de l'échantillon et ne s'étend pas jusqu'au capteur d'image. Cette configuration présente cependant l'inconvénient d'une interface air/liquide d'imprégnation non plane et mal maîtrisée, par rapport aux configurations illustrées sur les figures 1A et 1B.

Le recours à un liquide d'imprégnation entre un échantillon et un capteur d'images est connu de l'art antérieur, et en particulier dans la publication de W. Luo précédemment citée. Cependant, un élément important de l'invention est que l'échantillon 10 est placé au contact du liquide d'imprégnation 25 et s'imprègne de ce dernier, contrairement à l'art antérieur, selon lequel le liquide d'imprégnation s'étend jusqu'à une lame transparente délimitant l'échantillon, et n'est pas directement en contact avec l'échantillon. L'imprégnation du liquide d'imprégnation 25 dans l'échantillon 10 modifie son aspect visuel en augmentant sa transparence, en réduisant la diffusion de l'onde lumineuse incidente $12_i$ dans l'échantillon. L'invention tire profit que le liquide d'imprégnation 25 pénètre dans l'échantillon 10, ce dernier étant poreux.

Les images acquises sur le capteur d'image 20 ne permettent pas d'obtenir une représentation suffisamment précise de l'échantillon observé, notamment lorsque ce dernier est une lame de tissu. Comme décrit en lien avec l'art antérieur, on peut appliquer, à chaque image *I* acquise par le capteur d'image, dans une bande spectrale λ, un opérateur de propagation h, de façon à calculer une grandeur représentative de l'onde lumineuse d'exposition 22 transmise par l'échantillon 10, et à laquelle est exposée le capteur d'image 20. Un tel procédé, désigné par le terme reconstruction holographique, permet notamment de reconstruire une image du module ou de la phase de cette onde lumineuse dans un plan parallèle au plan de détection $P_{20}$, et notamment dans le plan $P_0$ selon lequel s'étend l'échantillon. Pour cela, on effectue un produit de convolution entre une image *I* acquise par le capteur d'image 20, désignée par le terme hologramme, et un opérateur de propagation h. Il est alors possible de reconstruire une amplitude complexe $\alpha$ de l'onde lumineuse 22 en tout point de coordonnées (*x,y,z*) de l'espace, et en particulier dans un plan situé à une distance | *z* | du capteur d'image 20, ce plan pouvant être le plan de l'échantillon $P_0$. L'amplitude complexe $\alpha$ est une grandeur complexe dont l'argument et le module sont respectivement représentatifs de la phase et de l'intensité de l'onde lumineuse à laquelle est exposée le capteur d'image.

L'opérateur de propagation $h(x,y,z)$ a pour fonction de décrire la propagation de la lumière entre le capteur d'image 20 et un point de coordonnées (*x,y,z*) situé à une distance | *z* | du capteur d'image 20. Il est alors possible de déterminer le module $M(x,y,z)$ et/ou la phase $\varphi(x,y,z)$ l'onde lumineuse d'exposition 22, à cette distance | *z* |, dite distance de reconstruction, avec :

$$M(x,y,z) = abs\,[\alpha(x,y,z)]$$

$$\varphi(x,y,z) = arg\,[\alpha(x,y,z)]$$

Les opérateurs abs et arg désignent respectivement le module et l'argument.

L'expression complexe de l'onde lumineuse d'exposition 22, à une coordonnée (x,y,z) est donnée par $\alpha(x,y,z) = \sqrt{I(x,y)} * h(x,y,z)$ le symbole * désignant un produit de convolution. $\sqrt{I(x,y)}$ désigne la racine carrée de l'intensité des pixels de coordonnées (x, y) de l'image *I* acquise dans le plan de détection $P_{20}$, z désignant ici une distance de reconstruction par rapport au capteur d'image.

L'opérateur de propagation peut être par exemple la fonction de Fresnel-Helmholtz, telle que :

$$h(x,y,z) = \frac{1}{j\lambda z}\,e^{j2\pi\frac{z}{\lambda}}\exp(j\pi\frac{x^2+y^2}{\lambda z}).$$

où λ désigne la longueur d'onde.

Lorsque la distance de reconstruction z correspond à la distance d entre le capteur d'image 20 et le plan de l'échantillon $P_0$, on peut obtenir une image $I^0$ du module $M^0$ et/ou de la phase $\varphi^0$ de l'onde lumineuse d'exposition d'exposition 22 dans le plan de l'échantillon $P_0$, chacune de ces images, dites images reconstruites, constituant une représentation de cet échantillon 10. La suite de la description, relative à des essais expérimentaux montre que l'image représentant la phase de l'onde lumineuse d'exposition 22 dans le plan de l'échantillon $P_0$ comporte une information riche quant à l'échantillon, et peut être utilisée à des fins d'analyse histologique.

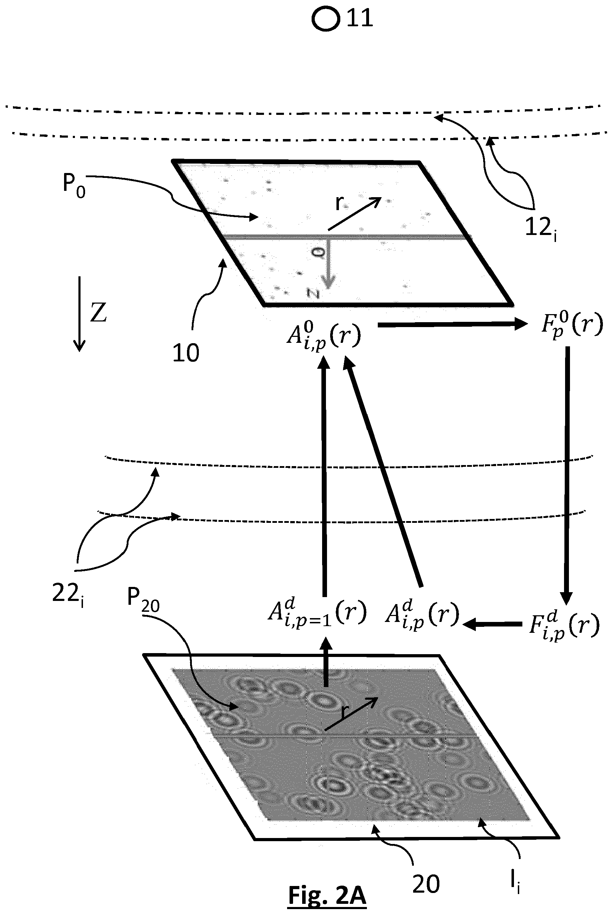

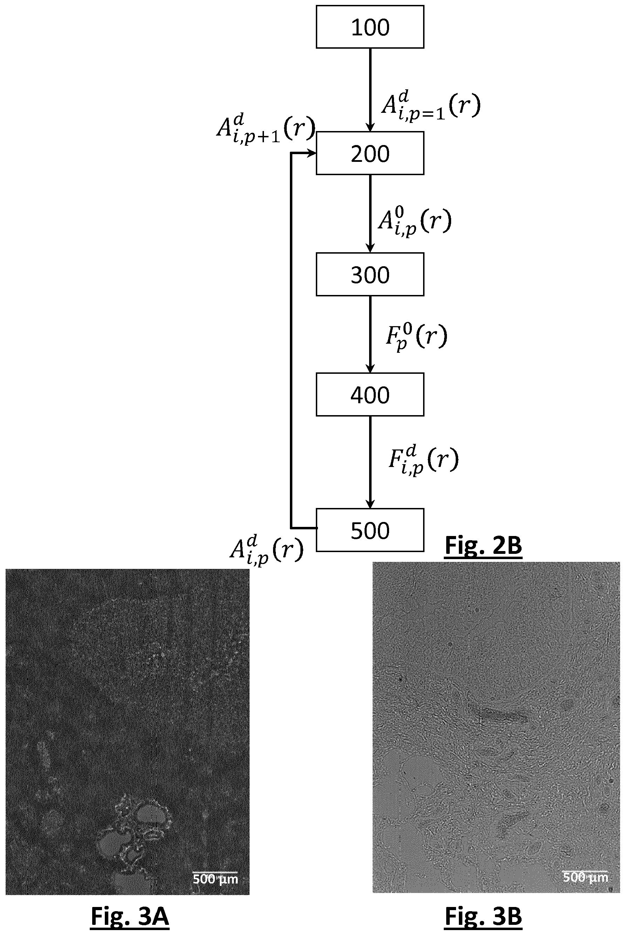

Les inventeurs ont développé un procédé de reconstruction holographique optimisé, mettant à profit l'acquisition d'images $I_i$ de l'échantillon dans plusieurs bandes spectrales $\lambda_i$. Pour cela, l'échantillon est éclairé simultanément en activant les trois sources de lumière élémentaires précédemment décrites $11_1$, $11_2$ et $11_3$. Le capteur d'image 20 acquiert une image *I*, à partir de laquelle trois images $I_1$, $I_2$, $I_3$ sont formées, respectivement selon les première, deuxième et troisième bande spectrale $\lambda_1$, $\lambda_2$, $\lambda_3$ respectivement associées auxdites sources de lumière élémentaires, comme précédemment décrit. Le procédé mis en oeuvre est un procédé itératif, comportant les étapes décrites ci-dessous, en lien avec les figures 2A et 2B. On pourra également se référer à la demande de brevet WO2016189257, en particulier entre les pages 11 et 22.

1^ère étape : initialisation

Lors d'une première étape 100 d'acquisition d'images, chaque source de lumière élémentaire $11_i$ de la source de lumière 11 est simultanément activée. Le capteur d'image acquiert une image *I*, à partir de laquelle le processeur forme des image $I_i$ correspondant respectivement à chaque bande spectrale $\lambda_i$, l'indice i, relatif à la bande spectrale, étant compris entre 1 et N, N étant le nombre de bandes spectrales considérées. Dans cet exemple, N = 3. La formation

de chaque image $I_i$ peut comprendre une étape dite de démosaïquation, comportant une interpolation de l'intensité entre les pixels exposés à une même bande spectrale.

L'échantillon est placé à une coordonnée axiale z = 0, selon l'axe de propagation Z. On désigne par r une coordonnée radiale, c'est-à-dire une coordonnée dans un plan perpendiculaire à l'axe de propagation Z. Le plan z = d correspond au plan de détection $P_{20}$, tandis que le plan z = 0 correspond au plan de l'échantillon $P_0$.

Si $I_i^{z=d}(r) = I_i^d(r)$ désigne la valeur de l'intensité collectée, dans la bande spectrale $\lambda_i$, par le pixel du capteur d'image de coordonnée radiale r dans le plan de détection $P_{20}$, il est possible d'établir, à l'aide de l'image $I_i$ une amplitude complexe $\alpha_i^{z=d}(r) = \alpha_i^d(r)$ de l'onde $22_i$ au niveau dudit pixel de coordonnée r, dont le module peut être exprimé selon l'expression :

$$M_i^d(r) = \sqrt{I_i^d(r)}$$

L'exposant d exprime le fait que l'amplitude complexe est déterminée selon le plan de détection $P_{20}$, d'équation z = d. Comme précédemment évoqué, l'amplitude complexe $\alpha_i^d(r)$ comporte un module et un argument, de telle sorte que :

$$\alpha_i^d(r) = M_i^d(r)e^{j\varphi_i^d(r)}$$

où :

- $M_i^d(r)$ désigne le module de l'amplitude complexe de l'onde lumineuse $22_i$ détectée par le capteur d'image, dans la i$^{\text{ème}}$ bande spectrale $\lambda_i$, à une coordonnée radiale r dans le plan de détection ;

- $\varphi_i^d(r)$ désigne la phase de l'amplitude complexe de l'onde lumineuse $22_i$ détectée par le capteur d'image, dans la i$^{\text{ème}}$ bande spectrale $\lambda_i$ et à ladite coordonnée radiale r dans plan de détection.

Or, le capteur d'images ne fournit aucune information relative à la phase de l'onde lumineuse $22_i$. Aussi, lors de l'étape 100, on considère que $e^{j\varphi_i^d(r)}$ est égal à une valeur initiale arbitraire, par exemple égale à 1.

L'amplitude complexe $\alpha_i^d(r)$ peut être exprimée de façon normalisée, selon l'expression :

$$A_i^d(r) = \frac{\alpha_i^d(r)}{\sqrt{I_i^{mean}}}$$

où :

- $I_i^{mean}$ désigne l'intensité moyenne d'une image formée dans la i$^{\text{ème}}$ bande spectale $\lambda_i$ ; cette intensité moyenne peut être déterminée expérimentalement, en disposant le le capteur d'image 20 face à la source de lumière 11, sans échantillon interposé entre l'un et l'autre, et en calculant la moyenne des pixels de l'image acquise par le capteur d'image 20 ;

- $A_i^d(r)$ désigne l'amplitude complexe normalisée de l'onde lumineuse $22_i$ détectée par le capteur d'image 20 dans la i$^{\text{ème}}$ bande spectrale $\lambda_i$.

La normalisation peut également être réalisée en divisant l'amplitude complexe $\alpha_i^d(r)$ par $I_i^{mean}(r)$, ou sa racine carrée, ce terme représentant l'intensité lumineuse, à la coordonnée radiale r, mesurée en l'absence d'échantillon.

L'amplitude complexe normalisée $A_i^d(r)$ comporte un module et un argument, de telle sorte que :

$$A_i^d(r) = m_i^d(r)e^{j\varphi_i^d(r)} \quad \text{où :}$$

- $m_i^d(r)$ désigne le module de l'amplitude complexe normalisée $A_i^d(r)$ dans le plan de détection ;

- $\varphi_i^d(r)$ désigne la phase de l'amplitude complexe normalisée, qui est également la phase de l'amplitude complexe $\alpha_i^d(r)$, dans le plan de détection.

La première étape 100 permet d'attribuer à chaque amplitude complexe $\alpha_i^d(r)$ ou à chaque amplitude complexe normalisée $A_i^d(r)$ une valeur initiale à partir de l'image $I_i$ acquise par le capteur d'image 20 dans la i$^{ème}$ bande spectrale $\lambda_i$, de telle sorte que :

$$\alpha_{i,p=1}^d(r) = M_i^d(r) = \sqrt{I_i^d(r)}$$

ou

$$A_{i,p=1}^d(r) = m_i^d(r) = \sqrt{\frac{I_i^d(r)}{I_i^{mean}}}$$

L'indice p correspond le rang de l'itération du procédé itératif décrit par la suite. L'étape 100 étant une étape d'initialisation, on attribue la valeur 1 à cet indice.

En adressant tout ou partie des pixels du capteur d'image 20, on obtient une image complexe, ou champ complexe, de l'onde lumineuse d'exposition 22$_i$ au niveau du plan de détection P$_{20}$, cette image rassemblant les amplitudes complexes $\alpha_i^d(r)$ ou les amplitudes complexes normalisées $A_i^d(r)$.

Dans la suite de la description, on ne considère que l'amplitude complexe normalisée $A_i^d(r)$, sachant que le raisonnement s'applique également à l'amplitude complexe $\alpha_i^d(r)$.

Cette première étape est répétée pour chaque bande spectrale ($\lambda_1...\lambda_N$) détectée par le capteur d'image 20.

2$^{ième}$ étape : rétro-propagation dans le plan P$_{10}$ de l'échantillon

Durant une deuxième étape 200, on estime l'amplitude complexe normalisée $A_{i,p}^0(r)$ de l'onde lumineuse d'exposition 22$_i$ à laquelle est exposé le capteur d'image, dans le plan de l'échantillon P$_0$. Cette estimation est réalisée par rétro-propagation de l'amplitude complexe normalisée $A_{i,p}^d(r)$ déterminée dans le plan de détection P$_{20}$, cette rétropropagation étant effectuée du plan de détection P$_{20}$ vers le plan de l'échantillon P$_0$.

Lors de la première itération (p = 1), on utilise l'amplitude complexe normalisée $A_{i,p=1}^d(r) = A_1^d(r)$ obtenue suite à la première étape 100. Lors des itérations suivantes (p > 1), on utilise l'amplitude complexe résultant de l'itération précédente, comme cela sera détaillé par la suite.

Comme précédemment décrit, en effectuant un produit de convolution entre l'amplitude complexe de l'onde lumineuse 22$_i$, relative à la bande spectrale $\lambda_i$, déterminée dans le plan de détection $z = d$, et un opérateur de propagation $h$, il est possible de reconstruire une amplitude complexe de cette même onde lumineuse en tout point de coordonnées (*r,z*) de l'espace, et en particulier dans le plan $P_0$ de l'échantillon. Autrement dit, l'amplitude complexe normalisée $A_{i,p}^z(r)$ de l'onde lumineuse $22_i$ peut s'obtenir, à un point de coordonnées (*r,z*), à partir de $A_{i,p}^{z=d}(r)$, selon l'opération :

$$A_{i,p}^z(r) = A_{i,p}^{z=d}(r) * h_{\lambda_i}(r, z - d),$$

Où $h_{\lambda_i}$ désigne l'opérateur de propagation h dans la bande spectrale $\lambda_i$. L'opérateur de propagation peut notamment reposer sur le modèle de diffraction de Fresnel. Dans cet exemple, l'opérateur de propagation est la fonction de Fresnel-Helmholtz, telle que :

$$h(r, z) = \frac{1}{j\lambda_i z} e^{j2\pi \frac{z}{\lambda}} \exp(j\pi \frac{r^2}{\lambda_i z}).$$

où λ désigne la longueur d'onde.
Ainsi,

$$A_{i,p}^{z=0}(r) = A_{i,p}^0(r) = A_{i,p}^{z=d}(r) * h_{\lambda i}(r, -d)$$

$$= -\frac{1}{j\lambda_i d} e^{-j2\pi \frac{d}{\lambda_i}} \iint A_{i,p}^d(r') \exp -(j\pi \frac{(r - r')^2}{\lambda_i d}) dr'$$

où

- *r'* désigne les coordonnées radiales dans le plan $P_{20}$ du capteur d'image, d'équation *z = d,*
- *r* désigne les coordonnées radiales dans le plan de l'échantillon $P_0$, qui est ici le plan de reconstruction, d'équation *z = 0,*
- $\lambda_i$ désigne la longueur d'onde centrale de la bande spectrale considérée.

Lorsque la reconstruction s'effectue selon le sens la propagation de la lumière, par exemple de l'échantillon 10 vers le capteur d'image 20, on parle de propagation. Lorsque la reconstruction s'effectue selon le sens inverse de la propagation de la lumière, par exemple du capteur d'image 20 vers l'échantillon 10, on parle de rétro-propagation. $A_{i,p}^0(r)$ est donc obtenu par rétropropagation de $A_{i,p}^d(r)$ selon la distance d séparant le plan de détection $P_{20}$ du plan de l'échantillon $P_0$.

Cette deuxième étape est répétée pour chaque bande spectrale ($\lambda_1...\lambda_N$) émise par la source de lumière 11 ou, de façon plus générale, pour chaque bande spectrale ($\lambda_1...\lambda_N$) respectivement associée à chaque image ($I_1...I_N$) détectée par le capteur d'image 20.

Il est possible, à ce stade, d'établir une image du module ou de la phase de l'amplitude complexe $A_{i,p}^0(r)$ de chaque onde lumineuse $22_i$, dans le plan de l'échantillon $P_0$, l'amplitude complexe étant normalisée ou non, en calculant la valeur de $A_{i,p}^0(r)$ aux différentes coordonnées *r* dans le plan de l'échantillon $P_0$.

Chaque image du module de l'amplitude complexe $A_{i,p}^0(r)$ est représentative de l'intensité de l'onde lumineuse dans le plan de l'échantillon $P_0$, tandis que chaque image de l'argument de l'amplitude complexe $A_{i,p}^0(r)$ est représentative de la phase $\varphi_i^0$ de l'onde lumineuse $22_i$ dans le plan $P_0$ de l'échantillon. L'amplitude complexe normalisée $A_{i,p}^0(r)$ correspond à une fonction de transmission de l'onde incidente $12_i$ par l'échantillon 10 à la coordonnée radiale r.

3ième étape : détermination de la fonction de pondération

Au cours de l'étape 300, on constitue, dans le plan de l'échantillon P$_0$, une fonction de pondération, notée $F_p^0(r)$, de l'amplitude complexe de l'onde lumineuse transmise par l'échantillon dans les différentes bandes spectrales $\lambda_i$ considérées. Selon cet exemple, la fonction de pondération $F_p^0(r)$, dans le plan de l'échantillon est commune à chaque bande spectrale. Elle est obtenue en combinant les amplitudes complexes normalisées $A_{i,p}^0(r)$ de l'onde lumineuse transmise par l'échantillon, dans le plan P$_0$ de l'échantillon et dans les différentes bandes spectrales $\lambda_i$. Dans cet exemple, elle est obtenue par une somme pondérée de chaque amplitude complexe déterminée lors de l'étape 200, dans le plan P$_0$ de l'échantillon, selon l'expression :

$$F_p^0(r) = \frac{1}{\sum_i k_i} \sum_i k_i A_{i,p}^0(r)$$

où $k_i$ désigne un facteur de pondération associé à la i$^{\text{ème}}$ bande spectrale $\lambda_i$.

Les facteurs de pondération peuvent être égaux les uns aux autres, par exemple égaux à 1/3.

4ième étape : propagation de la fonction de pondération dans le plan du capteur d'image

L'étape 400 vise à propager, du plan de l'échantillon P$_0$ vers le plan du capteur d'image P$_{20}$, la fonction de pondération $F_p^0(r)$, déterminée lors de l'étape précédente. L'opérateur de propagation *h* étant dépendant de la longueur d'onde, cette propagation est effectuée pour chaque bande spectrale $\lambda_i$ considérée.

Ainsi, pour chaque bande spectrale $\lambda_i$, $F_{i,p}^d(r) = F_p^0(r) * h_{\lambda_i}(r, z = d)$.

Lorsque l'opérateur de propagation est un opérateur de Fresnel-Helmholtz tel que précédemment défini,

$$F_{i,p}^d(r) = \frac{1}{j\lambda_i d} e^{j2\pi\frac{d}{\lambda_i}} \iint F_{i,p}^0(r') \exp(j\pi \frac{(r-r')^2}{\lambda_i d}) dr'$$

L'opérateur de propagation dépendant de la longueur d'onde, on détermine autant de fonctions de pondération, dans le plan de détection, que de bandes spectrales considérées.

- *r'* désigne les coordonnées radiales dans le plan P$_{20}$ du capteur d'image, d'équation *z = d,*
- *r* désigne les coordonnées radiales dans le plan de l'échantillon P$_0$, qui est ici le plan de reconstruction, d'équation z = 0,
- $\lambda_i$ désigne la longueur d'onde centrale de la bande spectrale considérée.

5ième étape : Mise à jour de l'amplitude complexe dans le plan du capteur d'image

Dans l'étape 500, on utilise la valeur de la fonction de pondération $F_{i,p}^d(r)$, dans le plan de détection z = d, pour mettre à jour, dans le même plan, l'estimation de l'amplitude complexe normalisée $A_{i,p}^d(r)$ de l'onde lumineuse 22$_i$, à laquelle est exposé le capteur d'image 20, dans la bande spectrale $\lambda_i$.

La formule de mise à jour est :

$$A_{i,p}^d(r) = m_i^d(r) \times \frac{F_{i,p}^d(r)}{\left| F_{i,p}^d(r) \right|} = m_i^d(r) \times e^{j\tilde{\varphi}_{i,p}^d(r)}$$

où :

- $\left|F_{i,p}^{d}(r)\right|$ désigne le module de $F_{i,p}^{d}(r)$ ;

- $m_i^d(r)$ désigne le module de l'amplitude complexe initiale normalisée $A_{i,p=1}^d$ déterminé, à partir de chaque image $I_i$, lors de la première étape 100. Ce terme a une fonction d'attache aux données mesurées ;

- $\tilde{\varphi}_{i,p}^d$ désigne une estimation de la phase de l'amplitude complexe de l'onde $22_i$ dans la $i^{\text{ème}}$ bande spectrale $\lambda_i$ ;

- $A_{i,p}^d(r)$ désigne l'amplitude complexe de l'onde lumineuse $22_i$ transmise par l'échantillon 10, dans le plan du le capteur d'image 20, cette amplitude complexe constituant la base de l'itération suivante, de rang $p + 1$.

A la suite de cette étape, une nouvelle itération peut commencer, la donnée d'entrée de cette nouvelle itération de rang $p + 1$ étant $A_{i,p+1}^d(r) = A_{i,p}^d(r)$, cette nouvelle itération débutant par la rétro-propagation de chaque amplitude complexe normalisée $A_{i,p+1}^d(r)$, pour les différentes bandes spectrales considérées, dans le plan $P_0$ de l'échantillon, selon l'étape 200.

Les étapes 200 à 500 sont réalisées de manière itérative, soit selon un nombre d'itérations $p_{\max}$ prédéterminé, soit jusqu'à l'atteinte d'un critère de convergence, ce dernier pouvant être, par exemple, exprimé sous la forme d'un écart entre l'estimation de deux mêmes grandeurs entre deux itérations successives.

A l'issue du procédé, on dispose d'une estimation de l'amplitude complexe de l'onde lumineuse $22_i$, transmise par l'échantillon, et à laquelle est exposé le capteur d'image 20, dans le plan de détection $P_{20}$, d'équation $z = d$ et/ou dans le plan de l'échantillon $P_0$, d'équation $z = 0$, et cela pour chaque bande spectrale considérée. En utilisant les différentes amplitudes complexes reconstruites $A_{i,p}^0(r)$ dans le plan de l'échantillon $P_0$, on obtient une représentation précise de ce dernier, dans chacune des bandes spectrales considérées, en particulier en formant des images à partir du module ou de la phase desdites amplitudes complexes.

Cet algorithme, bien que décrit en relation avec une amplitude complexe normalisée $A_i$, s'applique également à l'amplitude complexe non normalisée $\alpha_i$.

A partir de l'amplitude complexe obtenue dans le plan de l'échantillon dans les différentes bandes spectrales $\lambda_i$, il est possible d'établir une image $I_i^0$ représentant :

- soit l'intensité, dans le plan $P_0$ de l'échantillon, de l'onde $22_i$ transmise par l'échantillon dans une bande spectrale $\lambda_i$ ;
- soit la phase $\varphi_i^0$, dans le plan $P_0$ de l'échantillon, de l'onde $22_i$ transmise par l'échantillon dans une bande spectrale $\lambda_i$.

Il est également possible de combiner les modules ou les arguments de l'amplitude complexe $A_{i,p}^d$, dans différentes bandes spectrales $\lambda_i$, par exemple sous la forme d'une moyenne, de façon à former une image $I^0$ représentant l'intensité ou la phase $\varphi^0$ de l'onde lumineuse 22 d'exposition transmise par l'échantillon 10, et à laquelle est exposée le capteur d'image 20.

<u>Essais réalisés.</u>

Des essais ont été réalisés en utilisant un échantillon obtenu à partir d'une biopsie d'un colon humain. Suite à la biopsie, le tissu a été immergé dans du formaldéhyde, ayant une fonction d'agent fixateur, puis déshydraté en le plongeant dans des solutions comportant une concentration croissante en alcool. Il a ensuite été inclus dans une matrice de paraffine puis découpé selon de fines lamelles d'épaisseur comprise entre 3 et 4 $\mu$m. Lors de ces essais, le liquide d'imprégnation 25 utilisé est une huile à immersion Olympus, dont l'indice de réfraction s'élève à 1.516 à 23°C. La lamelle de paraffine, formant l'échantillon test $10_{\text{test}}$, a ensuite été déposée sur une lame support 15, et une goutte de liquide d'imprégnation 25 a été appliquée sur la lamelle. L'échantillon 10 a ensuite été rapproché du capteur d'image 20 précédemment décrit, de telle sorte que le liquide d'imprégnation s'étende entre ce capteur et l'échantillon, selon la configuration illustrée sur la figure 1B.

Un autre lamelle de tissu n'a pas été mouillée par le liquide d'imprégnation et a également été observée, de façon à constituer un échantillon de référence $10_{ref}$.

Les figures 3A et 3B représentent une image acquise par le capteur d'image 20, désignée par le terme hologramme, en considérant respectivement l'échantillon de référence $10_{ref}$ et l'échantillon test $10_{test}$. L'image acquise est ici une image dans la première bande spectrale $\lambda_1$ (en l'occurrence 450 nm - 465 nm), représentée ici en niveaux de gris. On observe que l'image de l'échantillon de test (figure 3B) est nettement moins contrastée que l'image de l'échantillon de référence (figure 3A), l'échantillon de test ayant été rendu transparent par le liquide d'imprégnation 25.

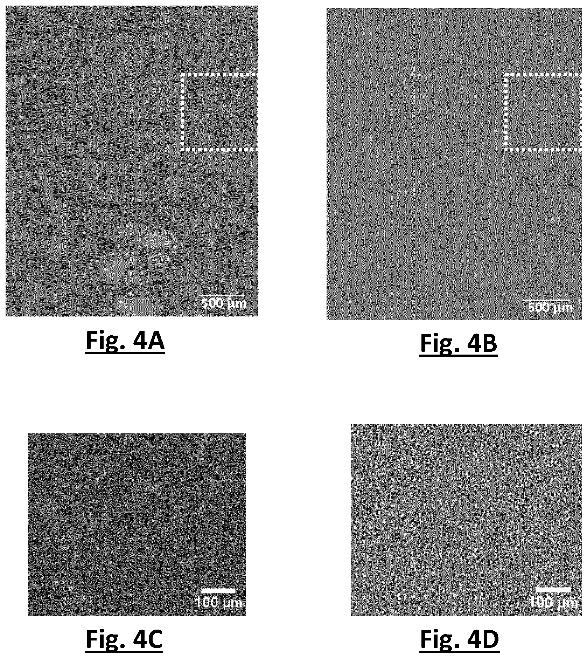

Les figures 4A et 4B représentent les résultats de reconstructions holographiques obtenues à partir de l'hologramme de l'échantillon de référence $10_{ref}$ illustré sur la figure 3A, en mettant en œuvre l'algorithme de reconstruction décrit en lien avec les figures 2A et 2B. La figure 4A représente le module $m_{i=1}^{0}(r)$ de l'amplitude complexe normalisée $A_{i=1}^{0}(r)$, dans le plan de l'échantillon $P_0$, tandis que la figure 4B représente la phase $\varphi_{i=1}^{0}(r)$ de l'amplitude complexe normalisée $A_{i=1}^{0}(r)$ dans le plan de l'échantillon $P_0$. Aucune de ces figures n'est vraiment exploitable.

Les figures 4C et 4D correspondent respectivement à des détails des images 4A et 4B, dans les régions d'intérêt délimitées par un cadre en pointillés.

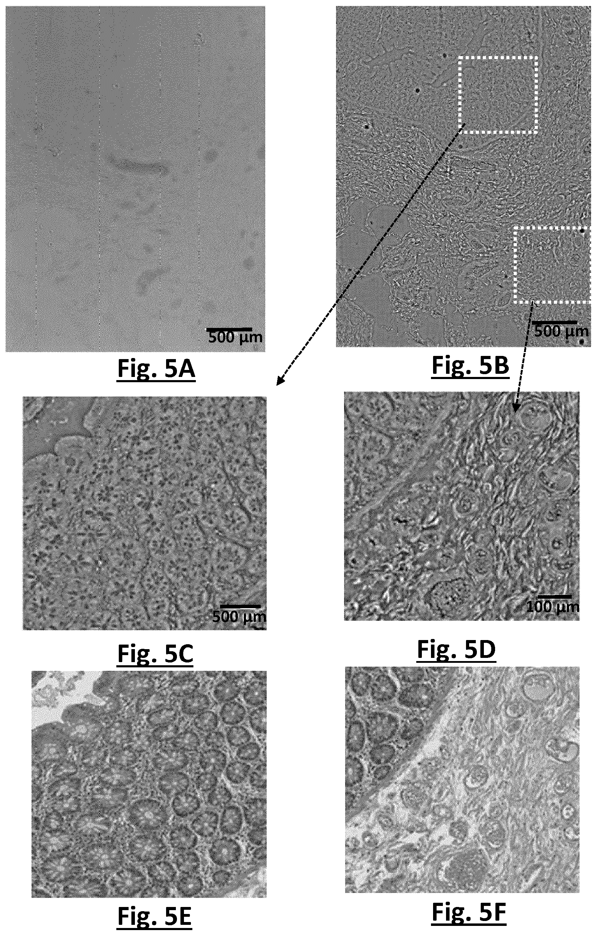

Les figures 5A et 5B représentent les résultats de reconstructions holographiques obtenues à partir de l'hologramme de l'échantillon de test $10_{test}$ illustré sur la figure 3B. La figure 5A représente le module $m_{i=1}^{0}(r)$ de l'amplitude complexe normalisée $A_{i=1}^{0}(r)$, dans le plan de l'échantillon $P_0$, tandis que la figure 5B représente la phase $\varphi_{i=1}^{0}(r)$ de l'amplitude complexe normalisée $A_{i=1}^{0}(r)$ dans le plan de l'échantillon $P_0$. La figure 5A ne comporte pas d'information exploitable, ce qui est attendu vu que l'échantillon de test $10_{test}$ a été rendu transparent par le liquide d'imprégnation 25. En revanche, la figure 5B est particulièrement riche en information et constitue une représentation exploitable de l'échantillon observé. Les figures 5C et 5D correspondent à des détails de l'image 5B, dans les régions d'intérêt délimitées, sur la figure 5B, par un cadre en pointillés. Le procédé de reconstruction mis en œuvre est rapide: la durée de l'acquisition s'élève à 0.5 seconde, tandis que la durée de la reconstruction est de l'ordre de la seconde, soit 1.5 seconde pour obtenir une image exploitable, le champ d'observation étant de 9.7 mm$^2$.

L'échantillon de test $10_{test}$ a été observé par microscopie après coloration HES, acronyme de Hématéine - Eosine - Safran, coloration courante dans le domaine de l'histologie. Les figures 5E et 5F représentent des images, correspondant sensiblement aux régions d'intérêt respectivement observées sur les figures 5C et 5D. La cohérence entre l'image de phase reconstruite et l'image obtenue au microscope est manifeste, ce qui atteste de la fiabilité et de l'intérêt de l'invention.

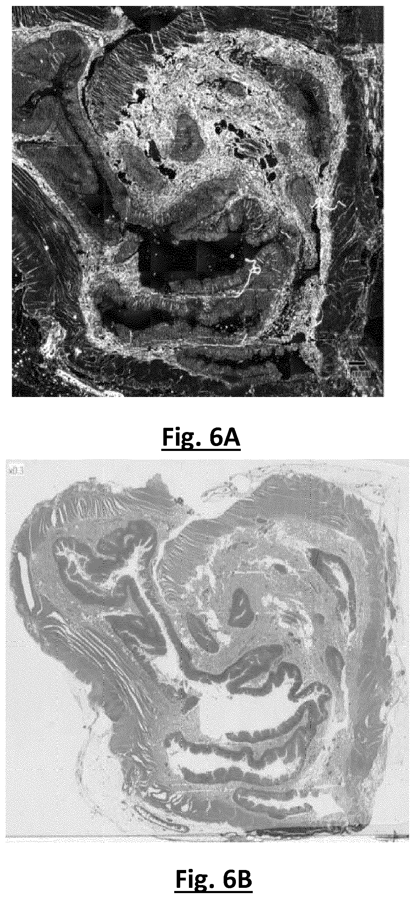

La position relative de l'échantillon de test $10_{test}$ et du capteur d'image 20 a été modifiée, grâce à la platine de translation 40. On a obtenu, par balayage, une représentation de l'ensemble de l'échantillon de test. La figure 6A représente une image de la phase $\varphi_{i=1}^{0}(r)$ de l'amplitude complexe normalisée $A_{i=1}^{0}(r)$, dans la première bande spectrale $\lambda_1$, reconstruite dans le plan de l'échantillon $P_0$, après application d'un filtre passe-haut, de type filtre de variance. Un tel filtre permet de calculer la valeur de la variance dans le voisinage de chaque pixel, la valeur du pixel étant remplacée par la valeur de la variance ainsi calculée. Le voisinage de chaque pixel peut être par exemple une zone de 5 par 5 pixels. D'autres filtres, de type passe haut, par exemple des filtres utisés pour la détection de contour, seraient utilisables. La figure 6B représente une observation de l'échantillon de test au microscope classique, ce qui constitue une méthode de référence. La cohérence entre ces deux images est élevée.

D'une manière générale, une image de la phase de l'onde transmise par l'échantillon, c'est-à-dire de l'onde à laquelle est exposé le capteur d'image, représente une distribution spatiale de l'indice de réfraction de l'échantillon, et cela dans la mesure où l'épaisseur de l'échantillon est considérée comme constante, ce qui est le cas lors de l'observation de lamelles de tissus biologiques.

Le procédé décrit ci-dessus permet d'obtenir une représentation d'un échantillon exploitable à pour l'histologie et l'aide au diagnostic, et cela en apportant les avantages suivants :

- par rapport à un microscope, le champ d'observation est plus élevé, ce qui permet l'observation d'un échantillon de grande surface, bien plus rapidement :
- le procédé ne comporte pas de coloration de l'échantillon ;
- par rapport à d'autres méthodes holographiques, l'imprégnation d'un échantillon par un liquide d'imprégnation permet d'obtenir des hologrammes dont la qualité est suffisante pour obtenir des images reconstruites permettant une caractérisation correcte, et rapide de l'échantillon.

L'invention pourra être appliquée pour l'observation d'un échantillon tissulaire en histologie mais également, en dehors de la biologie, d'un échantillon poreux et dont la transparence est augmentée sous l'effet d'une imprégnation par le liquide d'imprégnation. Les applications visées peuvent être dans les domaines de l'agroalimentaire, l'analyse d'échantillons industriels, par exemple des filtres, ou dans le domaine du contrôle de l'environnement.

**Revendications**

1. Procédé d'observation d'un échantillon poreux (10), comportant les étapes suivantes :

   i) illumination dudit échantillon à l'aide d'une source de lumière (11), apte à émettre une onde lumineuse incidente (12) se propageant vers l'échantillon (10);
   ii) acquisition, à l'aide d'un capteur d'image (20) s'étendant selon un plan de détection ($P_{20}$), d'au moins une image ($I$, $I_i$) de l'échantillon, l'échantillon étant disposé entre la source de lumière (11) et le capteur d'image (20), chaque image étant représentative d'une onde lumineuse d'exposition (22, 22i) transmise par l'échantillon sous l'effet de ladite illumination ;
   iii) application d'un opérateur de propagation à l'image acquise ($I$, $I_i$), lors de l'étape ii), de façon à former une image ($I^0$, $I_i^0$) représentative de ladite onde lumineuse (22, 22$_i$) transmise par l'échantillon, dans un plan ($P_0$) passant par ledit échantillon ;

   le procédé dans lequel l'échantillon est une lamelle de tissu biologique, et qui comprend également, préalablement à l'étape ii), l'application d'un fluide dit d'imprégnation (25) sur l'échantillon (10), de telle sorte que l'échantillon s'imprègne dudit liquide d'imprégnation, ledit liquide d'imprégnation ayant un indice de réfraction strictement supérieur à 1, de telle sorte que l'échantillon (10) est rendu transparent ou translucide après l'application du liquide d'imprégnation, et dans lequel l'image ($I^0$,$I_i^0$) formée lors de l'étape iii), représentative de l'onde lumineuse (22, 22$_i$) transmise par l'échantillon, est établie à partir de la phase ($\varphi^0$, $\varphi_i^0$), dans le plan ($P_0$) passant par l'échantillon, de ladite onde lumineuse (22, 22$_i$) transmise par l'échantillon.

2. Procédé selon l'une quelconque des revendications précédentes, dans lequel le liquide d'imprégnation (25) a un indice de réfraction compris entre 1.2 et 1.8 ou entre 1.3 et 1.7.

3. Procédé selon l'une quelconque des revendications précédentes, dans lequel le liquide d'imprégnation (25) s'étend de l'échantillon (10) jusqu'au capteur d'image (20).

4. Procédé selon la revendication 3, dans lequel le capteur d'image (20) comprenant une pluralité de pixels, le liquide d'imprégnation s'étend de l'échantillon (10) jusqu'auxdits pixels.

5. Procédé selon l'une quelconque des revendications 3 ou 4, dans lequel le liquide d'imprégnation (25) est appliqué sur l'échantillon (10) ou sur le capteur d'image (20), après quoi l'échantillon est rapproché du capteur d'image (20), jusqu'à ce que le liquide d'imprégnation s'étende de l'échantillon jusqu'au capteur d'image (20).

6. Procédé selon l'une quelconque des revendications 1 à 2, dans lequel une lame transparente (16) est disposée entre l'échantillon (10) et le capteur d'image (20), le liquide d'imprégnation (25) s'étendant entre l'échantillon et ladite lame transparente (16).

7. Procédé selon l'une quelconque des revendications précédentes dans lequel l'étape ii) comporte la formation d'une pluralité d'images ($I_i$), chaque image étant acquise dans une bande spectrale ($\lambda_i$) différente l'une de l'autre, l'étape iii) comportant alors les sous étapes suivantes :

   a) détermination, à partir de chaque image acquise ($I_i$) selon une bande spectrale ($\lambda_i$), d'une amplitude complexe initiale $(A_{i,p=1}^d(r))$ de l'onde lumineuse transmise (22$_i$), selon ladite bande spectrale, dans ledit plan de détection

(P$_{20}$) ;

b) à partir de chaque amplitude complexe $\left( A_{i,p=1}^d(r), A_{i,p}^d(r) \right)$ établie dans ledit plan de détection (P$_{20}$), selon une bande spectrale ($\lambda_i$), détermination d'une amplitude complexe $\left( A_{i,p}^0(r) \right)$ de l'onde transmise (22$_i$), selon chaque bande spectrale, dans le plan (P$_0$) selon lequel s'étend l'échantillon ;

c) combinaison d'une pluralité d'amplitudes complexes $\left( A_{i,p}^0(r) \right)$ déterminées lors de la sous-étape b), à différentes bandes spectrales, pour calculer une fonction de pondération $\left( F_p^0(r) \right)$, dans le plan de l'échantillon (P$_0$) ;

d) projection de ladite fonction de pondération $\left( F_p^0(r) \right)$ dans le plan de détection (P$_{20}$) de façon à obtenir, pour chaque bande spectrale ($\lambda_i$), une fonction de pondération $\left( F_{p,i}^d(r) \right)$ dans ledit plan de détection (P$_{20}$) ;

e) mise à jour de chaque amplitude complexe $\left( A_{i,p}^d(r) \right)$ de l'onde lumineuse (22$_i$) transmise, selon chaque bande spectrale ($\lambda_i$), dans le plan de détection (P$_{20}$), à l'aide de de ladite fonction de pondération $\left( F_{i,p}^d(r) \right)$ obtenue, dans ladite bande spectrale ($\lambda_i$), au cours de la sous-étape d) ;

f) répétition des sous-étapes b) à e) jusqu'à l'atteinte d'un critère d'arrêt.

8. Procédé selon la revendication 7, comportant une sous étape g) de formation d'une image (*I*, *I$_i$*) représentative de l'argument, dans le plan de l'échantillon (P$_0$), de l'amplitude complexe de l'onde (22$_i$) transmise par l'échantillon (10) selon au moins une bande spectrale ($\lambda_i$).

9. Procédé selon l'une quelconque des revendications précédentes, dans lequel aucune optique de grossissement n'est disposée entre l'échantillon (10) et le capteur d'image (20).

**Patentansprüche**

1. Verfahren zur Beobachtung einer porösen Probe (10), das die folgenden Schritte beinhaltet:

i) Beleuchten der Probe mithilfe einer Lichtquelle (11), die geeignet ist, eine einfallende Lichtwelle (12) auszusenden, die sich zur Probe (10) hin ausbreitet;

ii) Erfassen, mithilfe eines sich entlang einer Detektionsebene (P$_{20}$) erstreckenden Bildsensors (20), mindestens eines Bildes (I, I$_i$) der Probe, wobei die Probe zwischen der Lichtquelle (11) und dem Bildsensor (20) angeordnet ist, wobei jedes Bild für eine Belichtungslichtwelle (22, 22i) repräsentativ ist, die von der Probe unter der Wirkung der Beleuchtung durchgelassen wird;

iii) Anwenden eines Ausbreitungsoperators auf das beim Schritt ii) erfasste Bild (*I*, *I*i), so dass ein Bild (I$^0$, $I_i^0$) gebildet wird, das für die von der Probe durchgelassene Lichtwelle (22, 22$_i$) repräsentativ ist, in einer durch die Probe verlaufenden Ebene (P$_0$); wobei bei dem Verfahren die Probe eine Lamelle von biologischem Gewebe ist und das Verfahren auch, vor dem Schritt ii), das Applizieren eines sogenannten Imprägnierfluids (25) auf die Probe (10) umfasst, so dass die Probe mit der Imprägnierflüssigkeit imprägniert wird, wobei die Imprägnierflüssigkeit einen Brechungsindex strikt größer als 1 hat, so dass die Probe (10) nach dem Applizieren der Imprägnierflüssigkeit transparent oder transluzent gemacht wird, und wobei das beim Schritt iii) gebildete Bild (I$^0$, $I_i^0$), das für die von der Probe durchgelassene Lichtwelle (22, 22$_i$) repräsentativ ist, ausgehend von der Phase ($\varphi^0$, $\varphi_i^0$), in der durch die Probe verlaufenden Ebene (P$_0$), der von der Probe durchgelassenen Lichtwelle (22, 22$_i$) erstellt wird.

2. Verfahren nach einem der vorhergehenden Ansprüche, bei dem die Imprägnierflüssigkeit (25) einen Brechungsindex zwischen 1,2 und 1,8 oder zwischen 1,3 und 1,7 hat.

3. Verfahren nach einem der vorhergehenden Ansprüche, bei dem sich die Imprägnierflüssigkeit (25) von der Probe (10) bis zum Bildsensor (20) erstreckt.

4. Verfahren nach Anspruch 3, bei dem der Bildsensor (20) eine Vielzahl von Pixeln umfasst, wobei sich die Imprägnierflüssigkeit von der Probe (10) bis zu den Pixeln erstreckt.

5. Verfahren nach einem der Ansprüche 3 oder 4, bei dem die Imprägnierflüssigkeit (25) auf die Probe (10) oder auf den Bildsensor (20) appliziert wird, woraufhin die Probe dem Bildsensor (20) genähert wird, bis sich die Imprägnierflüssigkeit von der Probe bis zum Bildsensor (20) erstreckt.

6. Verfahren nach einem der Ansprüche 1 bis 2, bei dem ein transparentes Plättchen (16) zwischen der Probe (10) und dem Bildsensor (20) angeordnet wird, wobei sich die Imprägnierflüssigkeit (25) zwischen der Probe und dem transparenten Plättchen (16) erstreckt.

7. Verfahren nach einem der vorhergehenden Ansprüche, bei dem Schritt ii) das Bilden einer Vielzahl von Bildern ($I_i$) beinhaltet, wobei jedes Bild in einem anderen Spektralband ($\lambda_i$) erfasst wird, wobei Schritt iii) dann die folgenden Teilschritte beinhaltet:

a) Bestimmen, ausgehend von jedem gemäß einem Spektralband ($\lambda_i$) erfassten Bild ($I_i$), einer komplexen Ausgangsamplitude $\left(A_{i,p=1}^{d}(r)\right)$ der durchgelassenen Lichtwelle ($22_i$), gemäß dem Spektralband, in der Detektionsebene ($P_{20}$);

b) ausgehend von jeder komplexen Amplitude $\left(A_{i,p=1}^{d}(r)\right.$, $\left.A_{i,p}^{d}(r)\right)$, die in der Detektionsebene ($P_{20}$) gemäß einem Spektralband ($\lambda_i$) ermittelt wurde, Bestimmen einer komplexen Amplitude $\left(A_{i,p}^{0}(r)\right)$ der durchgelassenen Welle ($22_i$), gemäß jedem Spektralband, in der Ebene ($P_0$), entlang der sich die Probe erstreckt;

c) Kombinieren einer Vielzahl von im Teilschritt b) bestimmten komplexen Amplituden $A_{i,p}^{0}(r))$, mit verschiedenen Spektralbändern, um eine Gewichtungsfunktion $\left(F_p^{0}(r)\right)$ in der Ebene der Probe ($P_0$) zu berechnen;

d) Projizieren der Gewichtungsfunktion $\left(F_p^{0}(r)\right)$ in die Detektionsebene ($P_{20}$), so dass für jedes Spektralband ($\lambda_i$) eine Gewichtungsfunktion $\left(F_{p,i}^{d}(r)\right)$ in der Detektionsebene ($P_{20}$) erhalten wird;

e) Aktualisieren jeder komplexen Amplitude $\left(A_{i,p}^{d}(r)\right)$ der gemäß jedem Spektralband ($\lambda_i$) durchgelassenen Lichtwelle (22i) in der Detektionsebene ($P_{20}$) mithilfe der Gewichtungsfunktion $\left(F_{i,p}^{d}(r)\right)$, die in dem Spektralband ($\lambda_i$) während des Teilschritts d) erhalten wurde;
f) Wiederholen der Teilschritte b) bis e) bis zum Erreichen eines Stoppkriteriums.

8. Verfahren nach Anspruch 7, das einen Teilschritt g) des Bildens eines Bildes ($I$, $I_i$) beinhaltet, das für das Argument, in der Ebene der Probe ($P_0$), der komplexen Amplitude der von der Probe (10) gemäß mindestens einem Spektralband ($\lambda_i$) durchgelassenen Lichtwelle ($22_i$) repräsentativ ist.

9. Verfahren nach einem der vorhergehenden Ansprüche, bei dem keine Vergrößerungsoptik zwischen der Probe (10) und dem Bildsensor (20) angeordnet ist.

**Claims**

1. Method for observing a porous sample (10), comprising the following steps:

i) illuminating said sample using a light source (11), able to emit an incident light wave (12) that propagates towards the sample (10);

ii) acquiring, using an image sensor (20) lying in a detection plane ($P_{20}$), at least one image ($I$, $I_i$) of the sample, the sample being placed between the light source (11) and the image sensor (20), each image being representative of an exposure light wave (22, $22_i$) transmitted by the sample under the effect of said illumination;

iii) applying a propagation operator to the image ($I$, $I_i$) acquired in step ii), so as to form an image ($I^0$, $I_i^0$) representative of said light wave (22, $22_i$) transmitted by the sample, in a plane ($P_0$) passing through said sample;

wherein the sample is a thin section of biological tissue, the method also comprising, prior to step ii), applying a fluid (25) called the impregnation fluid to the sample (10), such that the sample is impregnated with said impregnation liquid, said impregnation liquid having a refractive index strictly higher than 1, such that the sample (10) is made transparent or translucent after the application of the impregnation liquid, and wherein the image ($I^0$, $I_i^0$) formed in step iii), which is representative of the light wave (22, $22_i$) transmitted by the sample, is established on the basis of phase ($\varphi^0$, $\varphi_i^0$), in the plane ($P_0$) passing through the sample, of said light wave (22, $22_i$) transmitted by the sample.

2. Method according to any one of the preceding claims, wherein the impregnation liquid (25) has a refractive index comprised between 1.2 and 1.8 or between 1.3 and 1.7.

3. Method according to any one of the preceding claims, wherein the impregnation liquid (25) extends from the sample (10) to the image sensor (20).

4. Method according to Claim 3, wherein, the image sensor (20) comprising a plurality of pixels, the impregnation liquid extends from the sample (10) to said pixels.

5. Method according to either one of Claims 3 and 4, wherein the impregnation liquid (25) is applied to the sample (10) or to the image sensor (20), after which the sample is brought closer to the image sensor (20), until the impregnation liquid extends from the sample to the image sensor (20).

6. Method according to either one of Claims 1 to 2, wherein a transparent slide (16) is placed between the sample (10) and the image sensor (20), the impregnation liquid (25) extending between the sample and said transparent slide (16).

7. The method according to any one of the preceding claims, wherein step ii) comprises forming a plurality of images ($I_i$), each image being acquired in a different spectral band ($\lambda_i$), step iii) then comprising the following substeps:

a) determining, from each image ($I_i$) acquired in a spectral band ($\lambda_i$), an initial complex amplitude $\left(A_{i,p=1}^d(r)\right)$ of the transmitted light wave (22i), in said spectral band, in said detection plane ($P_{20}$);

b) from each complex amplitude $\left(A_{i,p=1}^d(r), A_{i,p}^d(r)\right)$ established in said detection plane ($P_{20}$), in a spectral band ($\lambda_i$), determining a complex amplitude $\left(A_{i,p}^0(r)\right)$ of the transmitted wave ($22_i$), in each spectral band, in the plane ($P_0$) in which the sample lies;

c) combining a plurality of complex amplitudes $\left(A_{i,p}^d(r)\right)$ determined in substep b), in various spectral bands, to compute a weighting function $\left(F_p^0(r)\right)$, in the plane of the sample ($P_0$);

d) projecting said weighting function $\left(F_p^0(r)\right)$ into the detection plane ($P_{20}$) so as to obtain, for each spectral band ($\lambda_i$), a weighting function $\left(F_{p,i}^d(r)\right)$ in said detection plane ($P_{20}$);

e) updating each complex amplitude $\left(A_{i,p}^d(r)\right)$ of the light wave (22i) transmitted, in each spectral band ($\lambda_i$), in the detection plane ($P_{20}$), using said weighting function $\left(F_{i,p}^d(r)\right)$ obtained, in said spectral band ($\lambda_i$), in

substep d);
f) repeating substeps b) to e) until a termination criterion is met.

8. Method according to Claim 7, comprising a substep g) of forming an image ($I$, $I_i$) representative of the argument, in the plane of the sample ($P_0$), of the complex amplitude of the wave (22i) transmitted by the sample (10) in at least one spectral band ($\lambda_i$).

9. Method according to any one of the preceding claims, wherein no magnifying optic is placed between the sample (10) and the image sensor (20).

**Fig. 1A**

**Fig. 1B**

**Fig. 1C**

**Fig. 2A**

**Fig. 2B**

**Fig. 3A**

**Fig. 3B**

**Fig. 4A**

**Fig. 4B**

**Fig. 4C**

**Fig. 4D**

**Fig. 5A**

**Fig. 5B**

**Fig. 5C**

**Fig. 5D**

**Fig. 5E**

**Fig. 5F**

## Fig. 6A

## Fig. 6B

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2008090330 A
- US 2012142086 A
- US 20120218379 A
- WO 2012094523 A
- WO 2016189257 A

**Littérature non-brevet citée dans la description**

- **GREENBAUM A.** wide-field computational imaging of pathology slides using lensfree on-chip microscopy. *Sci. Transl. Med,* 2014, vol. 6, 267ra175
- **LUO W.** Synthetic aperturebased on-chip microscopy. *Light : Science & Applications,* 2015, vol. 4, e261
- **ZHU DAN.** Recent progress in tissue optical clearing. *Laser Photonics Rev.,* 2013, vol. 7 (5), 732-757
- **ZHERNOVAYA OLGA.** Study of optical clearing of blood by immersion method. *Dynamics and fluctuations in biomedical photonics VIII, proc of SPIE,* vol. 7898
- **ZHU D.** Recent Progress in Tissue Optical Clearing. *Laser&Photonics Reviews,* 2013, vol. 7 (5), 732-757